# FASCICULE DE BREVET EUROPEEN

(11) **EP 1 697 475 B1**
(45) Date de publication et mention de la délivrance du brevet: **09.04.2008**
(21) Numéro de dépôt: 04791503.8
(22) Date de dépôt: 08.10.2004
(51) Int. Cl.: C09D 131/04

(54) **COMPOSITIONS DE REVÊTEMENTS COMPRENANT UNE DISPERSION AQUEUSE DE POLYMÈRE FILMOGÈNE ET UNE SILICONE POLYETHER, LEUR PROCÉDÉ DE PRÉPARATION ET LEURS UTILISATIONS**
BESCHICHTUNGSZUSAMMENSETZUNG MIT EINER WÄSSRIGEN FILMBILDENDEN POLYMERDISPERSION UND EINEM POLYETHERSILICON, DEREN HERSTELLUNG UND VERWENDUNG
COATING COMPOSITIONS COMPRISING AN AQUEOUS FILM-FORMING POLYMER DISPERSION AND A POLYETHER SILICON, PREPARATION METHOD THEREOF AND USE OF SAME

(30) Priorité: 08.10.2003 FR 0311759
(43) Date de publication de la demande: 06.09.2006
(73) Titulaire: Hexion Specialty Chemicals Research Belgium S.A., 1348 Ottignies Louvain-la-Neuve (BE)
(72) Inventeur: LORENTZ, Gilles, F-69006 LYON (FR); JOUBERT, Daniel, F-60500 VINEUIL SAINT FIRMIN (FR)
(74) Mandataire: van der Straaten, Jan Anthony
(86) Numéro de dépôt international: PCT/FR2004/002553
(87) Numéro de publication internationale: WO 2005/035675

(56) Documents cités:
- EP-A- 0 679 699
- DE-A- 10 156 078
- GB-A- 1 521 156
- US-A- 3 337 351
- US-A- 3 933 407
- PATENT ABSTRACTS OF JAPAN vol. 0172, no. 43 (C-1058), 17 mai 1993 (1993-05-17) & JP 04 370176 A (NIPPON PAINT CO LTD), 22 décembre 1992 (1992-12-22)
- PATENT ABSTRACTS OF JAPAN vol. 2003, no. 08, 6 août 2003 (2003-08-06) & JP 2003 119397 A (DAINIPPON INK & CHEM INC), 23 avril 2003 (2003-04-23)

## Description

La présente invention concerne de nouvelles compositions de revêtements, ou de traitements de surface, notamment des compositions de peintures. Ces compositions peuvent être utilisées dans différentes applications telles que peintures à l'eau intérieures ou extérieures, crépis, vernis, revêtement semi-épais, formulations d'apprêts pour textiles architecturaux, surfaces plastique rigides ou souples, ou produits de protection de surface. Elles sont avantageusement employées pour réaliser des revêtements antisalissures.

L'art antérieur n'indique pas de composition de revêtement apte à filmifier correctement, qui confère à l'application finale, par exemple sous forme de peinture, une forte hydrophilie à la surface extérieure du revêtement qui soit durable , c'est-à-dire qui résiste aux lavages, sans réduire la capacité de protection du revêtement, c'est-à-dire sans détériorer la formation du film protecteur ou son adhérence sur le support recouvert.

L'un des objectifs essentiels de la présente invention est donc de proposer une nouvelle composition de revêtement conférant à l'application finale une forte hydrophilie à la surface extérieure du revêtement qui soit durable, c'est-à-dire qui résiste aux lavages, sans réduire la capacité de protection du revêtement, c'est-à-dire sans détériorer la formation du film protecteur ou son adhérence sur le support recouvert.

Plus concrètement, la nouvelle composition de revêtement, mise au point et faisant l'objet de la présente invention, comprend :
- une dispersion aqueuse de polymère filmogène, et
- une quantité suffisante d'une silicone polyether vérifiant la formule (I) suivante : 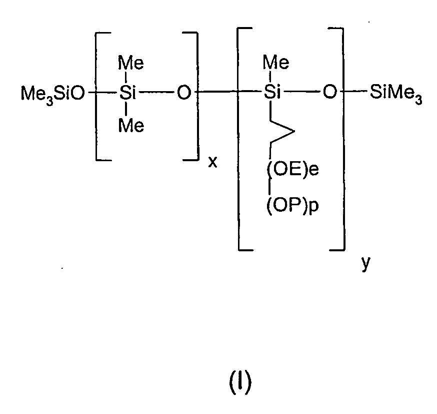
les groupes terminaux des oxydes d'éthylène ou de propylène étant des groupes OR
dans laquelle :
OE signifie -O-CH₂-CH₂-
OP signifie -O-CH₂-CH₂-CH₂-
R représente un atome d'hydrogène, ou un radical alkyle linéaire ou ramifié ayant de 1 à 22 atomes de carbone, et de préférence ayant de 1 à 4 atomes de carbone,
x est un nombre compris entre 5 et 50,
y est un nombre compris entre 3 et 10,
e est un nombre compris entre 10 et 30,
p est un nombre compris entre 0 et 10,
étant entendu que :
   x/y est inférieur à 10 et de préférence inférieur ou égal à 8,
   e+p est inférieur à 30 et de préférence inférieur ou égal à 20,
   e/p est supérieur à 1 et de préférence supérieur ou égal à 4, et
   x+y est inférieur à 60 et de préférence inférieur à 40.

Les formes préférées sont particulèrement les produits pour lesquels
x= 9,5, y=3,5, e=11,5 et p=2,5, et R est un atome d'hydrogène ;
x= 14, y= 4, e =17 et p= 1, et R est un atome d'hydrogène ;
x= 48, y = 6 , e =15 et p = 5, et R est un atome d'hydrogène.

Ces produits sont tous des produits liquides non aqueux.

Par dispersion aqueuse de polymère filmogène insoluble dans l'eau (latex) on entend les latex naturels ou synthétiques.

Les polymères filmogènes insolubles dans l'eau préférés sont obtenus par polymérisation de monomères choisis parmi :
- les esters vinyliques et plus particulièrement l'acétate de vinyle ;
- les acrylates et méthacrylates d'alkyle dont le groupe alkyle contient de 1 à 10 atomes de carbone par exemple les acrylates et méthacrylates de méthyle, éthyle, n-butyle, 2-éthylhexyle ;
- les monomères vinylaromatiques en particulier le styrène.
   Ces monomères peuvent être copolymérisés entre eux ou avec d'autres monomères à insaturation éthylénique, pour former des homopolymères, des copolymères ou des terpolymères.

A titre d'exemples non limitatifs de monomères copolymérisables avec l'acétate de vinyle et/ou les esters acryliques et/ou le styrène, on peut citer l'éthylène et les oléfines comme l'isobutène; les esters vinyliques d'acides monocarboxyliques saturés, ramifiés ou non, ayant de 1 à 12 atomes de carbone, comme le propionate, le "Versatate" (marque déposée pour les esters d'acides ramifiés en C₉-C₁₁), le pivalate, le laurate de vinyle ; les esters d'acides insaturés mono- ou di-carboxyliques possédant 3 à 6 atomes de carbone avec les alcanols possédant 1 à 10 atomes de carbone, comme les maléates, fumarates de méthyle, d'éthyle, de butyle, d'éthylhexyle ; les monomères vinylaromatiques tels que les méthylstyrènes, les vinyltoluènes ; les halogénures de vinyle tels que le chlorure de vinyle, le chlorure de vinylidène, les diolefines particulièrement le butadiène ; les esters (méth)allyliques de l'acide (meth)acrylique, les esters (méth)allyliques des mono et diesters des acides maléique, fumarique et itaconique, ainsi que les dérivés alkèniques des amides des acides acryliques et méthacryliques, tels que le N-méthallylmaléimide.

On peut notamment choisir au moins 2 monomères copolymérisables de natures différentes pour obtenir un terpolymère.
On peut citer à titre d'exemple un terpolymère de type acétate/versatate/dibutylmaléate.

La polymérisation du latex est réalisée de manière connue en soi en émulsion aqueuse des monomères polymérisables en présence d'au moins un initiateur radicalaire et de préférence d'un agent de transfert, par exemple du type mercaptan avec une concentration en monomères dans le milieu réactionnel comprise généralement entre 20 et 60 % en poids.

La polymérisation peut être réalisée en continu, en discontinu ou semi-continu avec introduction d'une partie des monomères en continu et être du type "ensemencé" ou "incrémentale" selon toute variante connue pour l'obtention des particules de structure homogène et hétérogène.

Pour la préparation du latex, à titre d'exemple non limitatif, on se référera aux modes opératoires décrits dans le brevet EP 599 676 au nom de la présente demanderesse.

D'une manière préférée on utilise des compositions de revêtements acryliques, c'est-à-dire qu'ils comprennent des polymères à base de monomères de type acryliques (tels que les acrylates et méthacrylates d'alkyle dont le groupe alkyle contient de 1 à 10 atomes de carbone, par exemple les acrylates et méthacrylates de méthyle, éthyle, n-butyle, 2-éthylhexyle). Ils peuvent comprendre également d'autres monomères ; il peut s'agir par exemple de vernis styrène-acrylique.
Cependant, dans les applications visées, les compositions de revêtements "purs" acryliques, c'est-à-dire à base de monomères de type acryliques seulement, sont encore plus préférées.

Les latex utilisés sont choisis de telle façon que leur température de transition vitreuse (TG) soit comprise entre 10 et 60 °C, et de préférence comprise entre 20 et 40 °C.
Les tailles des particules de polymère en dispersion aqueuse constituant les latex selon l'invention peuvent être situées entre 300 nanomètres et 20 nanomètres. Ces tailles de particules sont mesurées par granulo laser ou par microscopie à balayage après cryofracture de l'échantillon.

Eventuellement, les dispersions aqueuses de polymères filmogènes peuvent comprendre des plastifiants, ceci afin de diminuer la température de formation de film (TMMF) lorsque le procédé de l'invention est mis en oeuvre dans des conditions de température très froides, c'est-à-dire à des températures inférieures à 0 °C.

D'une manière générale, la quantité suffisante de silicone polyether de formule (I) ajoutée dans la dispersion aqueuse de polymère filmogène (latex) est comprise entre 0,1 et 10 % en poids de silicone polyether de formule (I) sèche par rapport au poids de latex sec.

De manière préférée, la quantité suffisante de silicone polyether de formule (I) ajoutée dans la dispersion aqueuse de polymère filmogène (latex) est comprise entre 1 et 5 % en poids de silicone polyether de formule (I) sèche par rapport au poids de latex sec.

L'ajout de silicone polyether de formule (I) doit bien sûr être convenablement dosé pour des questions économiques et aussi techniques.

L'ajout du silicone polyether de formule (I) est effectué par simple addition du silicone polyether liquide à la dispersion aqueuse de polymère filmogène (latex), c'est-à-dire par une technique de mélange liquide/liquide.

Le mélange du silicone polyether de formule (I) et de la dispersion aqueuse de polymère filmogène peut être effectué par mélange liquide/liquide dans tout mélangeur liquide/liquide permettant de mélanger sans provoquer d'introduction d'air.

L'utilisation d'une silicone polyether de formule (I) de l'invention dans des compositions de revêtements présente plusieurs avantages.

Tout d'abord ce composé est miscible, compatible et stable lorsqu'il est mélangé à la dispersion aqueuse de polymère filmogène (latex).
De plus, il présente l'avantage de ne pas empêcher ni modifier la filmification lors de l'application de la composition de revêtement sur un support.

Plus particulièrement, ce composé confère une forte hydrophilie à la surface du film formé sans réduire la capacité de protection du revêtement. Sans vouloir se limiter à une théorie scientifique ou à un mécanisme, il semble que cette propriété est due au fait que le silicone polyether, porteur des groupements hydrophilisants, est localisé, après formation du film, uniquement à l'extrême surface dudit film, du côté exposé à l'air.

De plus, ce composé confère durablement cet effet d'hydrophilie au revêtement, et n'est pas lessivé par les pluies ou les lavages.
Les mélanges de latex et de silicone polyether de formule (I) selon l'invention sont stables et restent homogènes même après stockage prolongé et y compris lorsqu'ils sont exposés à une température de 40 °C pendant 2 mois.

L'hydrophilie conférée à l'extrême surface par l'ajout de silicones polyether de formule (I) est évaluée par la méthode classique dite de l'angle de mouillage. Une goutte d'eau distillée de taille calibrée est déposée dans des conditions standardisées et répétitives sur la surface à étudier. Elle s'étale ou se rétracte plus ou moins selon l'hydrophilie de la surface, et avec un appareillage adapté, comme par exemple un gonionimétre de mesure de l'angle de mouillage, par exemple de marque Tantec, il est possible de mesurer l'angle formé par la goutte déposée et la surface.
Les angles de contact élevés supérieurs à 70° correspondent à des surfaces hydrophobes. Les angles inférieurs, en particulier ceux inférieurs à 40° (ce qui signifie qu'on obtient un étalement de la goutte d'eau) correspondent à des surfaces hydrophiles.
La durabilité de l'hydrophilie de surface conférée par le système selon l'invention est évaluée par la même technique, mais pratiquée sur des films ou des objets revêtus de ces films qui ont été totalement immergés dans de l'eau pure pendant plusieurs périodes de 12 heures, puis séchés à 50 °C pendant 1 heure.
Dans les exemples selon l'invention, l'hydrophilie de surface est toujours constatée même après 15 cycles d'immersion totale et séchage.

Outre les deux constituants principaux de la composition de revêtement de l'invention, celle-ci peut contenir également d'autres additifs tels que par exemple des agent(s) anti-mousse(s), biocide(s), tensio-actif(s), agent(s) rhéologique(s), agent(s) de coalescence, agent(s) dispersant(s), et agent(s) épaississant(s).

Mais de manière préférentielle, la composition utilisée est simplement l'association entre un latex et une silicone polyether selon l'invention, de formule (I) et elle est mise en oeuvre comme une couche de finition, par dessus la couche de revêtement classique.

Pour la préparation de la composition de revêtement, on mélange les différents constituants de façon connue en soi.

La composition de revêtement selon l'invention peut être appliquée selon les techniques habituelles. A titre d'exemple, elle peut être appliquée sur les surfaces par tout moyen convenable tel que pinceau, brosse, pulvérisateur, etc...

Les surfaces sur lesquelles la composition de revêtement selon l'invention est applicable sont de natures diverses.

Cependant afin d'obtenir une bonne hydrophilie à la surface extérieure du revêtement, il est important d'appliquer la composition de revêtement sur un support hydrophobe.

Les supports hydrophobes sont choisis parmi le verre, les métaux, le polypropylène rigide ou sous forme de voile non tissé, le bois traité par une première couche de latex selon l'invention mais sans silicone polyether, ou un matériau à base de ciment également traité par une première couche de latex seul également. Ils sont donc hydrophobes dans le sens ou le test de l'angle de contact avec une goutte d'eau donne des angles de mouillage de l'ordre de 70° ou supérieurs.

Il est donc important de noter que tout support y compris des supports non hydrophobes peut-être rendu hydrophobe au sens de la présente invention par enduction préalable par un primaire d'adhérence, c'est-à-dire une composition de polymère filmogène en dispersion aqueuse (latex) ou à base de solvants.

Par exemple des matériaux poreux à caractére hydrophile tel le bois brut, le ciment, les objets préfabriqués en ciment ou fibro-ciment, ou encore la brique ou les tuiles non vernissées, ou les baches textiles en coton tendu doivent être enduits par une composition de polymère filmogène en dispersion aqueuse ou à base de solvants (primaire d'adhérence hydrophobe) avant l'application de la composition de revêtement selon l'invention.

Ce primaire d'adhérence hydrophobe peut notamment être à base du latex qui est utilisé dans la composition de revêtement qui est appliquée ultérieurement.

L'invention a également pour objet un procédé pour rendre plus hydrophile (angle de mouillage avec l'eau de l'ordre de 30°) et de façon durable un support hydrophobe caractérisé en ce qu'on applique sur la surface du support hydrophobe une quantité suffisante d'une composition de revêtement selon l'invention.

La présente invention a également pour objet un support hydrophobe dont la surface est recouverte au moins en partie par un film provenant du séchage d'une composition comprenant une émulsion aqueuse de polymère filmogène et au moins une silicone polyether de formule (I).

Les compositions de l'invention sont utiles pour différentes applications.

De préférence, la composition de revêtement de l'invention est utilisée pour réaliser un revêtement antisalissure.

Ainsi, une super-hydrophilie à l'extrême surface du revêtement, celui-ci étant lui-même fermé (non poreux) et hydrophobe dans sa masse, permet d'éviter la fixation des salissures ou des micro-organismes. La super-hydrophilie à l'extrême surface du revêtement permet une meilleure lavabilité de la surface par l'eau de pluies ou un simple arrosage.

La composition de revêtement selon l'invention peut donc être utilisée dans un vernis ou une peinture extérieure mais également intérieure.

En effet, le problème d'ensalissement existe pour tous les éléments exposés à l'extérieur, comme par exemple les façades, les textiles dits architecturaux (bâches, toiles tendues, chapiteaux fixes), les surfaces métalliques peintes, le bois, mais également pour les éléments à l'intérieur, peintures intérieures lessivables pour cuisine ou salle de bain, les vernis de finition de papier peints, les surfaces carrelées vernissées, le bois, les éléments préfabriqués en ciment ou fibrociments par exemple.

Les salissures peuvent être des salissures grasses et carbonées, issues essentiellement de la pollution automobile ou des chauffages urbains. Il s'agit dans ce cas d'huiles, des suies, des mélanges d'huile et de particules de noir de carbone, des aérosols de fumées et de suies.
Mais les salissures peuvent être des salissures d'origines biologiques constitués de lichens, symbioses d'algues et de champignons.

On peut citer également à titre d'exemple l'utilisation d'une composition de revêtement de l'invention pour réaliser un revêtement plus hydrophile des polymères utilisés dans les couches culottes pour bébé.

Les exemples et tests suivants sont donnés à titre illustratif. Ils permettent notamment de mieux comprendre l'invention et de faire ressortir ses avantages et montrent quelques variantes de réalisation.

### Exemple 1

1 - On prépare des compositions par addition des silicones polyether candidats au latex , à l'ambiante, au taux de 3 %, exprimé en sec/sec par rapport au latex Le tableau I ci-dessous donne les compositions des produits testés selon la formule (I) donnée précédemment.
   Deux produits Rhodorsil figurent dans ce tableau et sont des produits commerciaux vendus par la société Rhodia.
   Les autres produits sont des produits obtenus à l'échelle du laboratoire.
   Le latex objet des essais est le Rhodopas D2040, latex acrylique commercialisé par la société Rhodia.
2 - On observe les effets sur l'émulsion et sur la stabilité dans le temps
3 - Pour les mélanges stables, on prépare un "film" de 2 mm d'épaisseur dans une empreinte silicone. Ceci permet d'extraire le film facilement et de pratiquer sur ce film divers tests ou mesures
4 - On mesure l'hydrophilie des deux faces (celle ayant été exposée à l'air et celle non exposée), par mesure de l'angle de mouillage d'une goutte d'eau permutée déposée en surface, comme précisé précédemment.

Le tableau I ci-dessous donne la définition des silicones polyethers objets des essais.

**Tableau I**

| | **Référence des produits** | **Origine** | **x** | **y** | **e** | **p** | **R** |
|---|---|---|---|---|---|---|---|
| Exemple comparatif | Silicone polyether 1 | Rhodorsil SP3300 | 75 | 7 | 22 | 22 | H |
| Exemple comparatif | Silicone polyether 2 | Produit de laboratoire | 31 | 6 | 29 | 48 | H |
| Exemple comparatif | Silicone polyether 3 | Produit de laboratoire | 29 | 4,5 | 5,7 | 32,6 | H |
| Exemple de l'invention | Silicone polyether 4 | Produit de laboratoire | 14 | 4 | 17 | 1 | H |
| Exemple de l'invention | Silicone polyether 5 | Produit de laboratoire | 20 | 4 | 22 | 7 | H |
| Exemple de l'invention | Silicone polyether 6 | Produit de laboratoire | 19 | 5 | 25 | 4 | H |
| Exemple de l'invention | Silicone polyether 7 | Produit de laboratoire | 21 | 4 | 20 | 5 | H |
| Exemple de l'invention | Silicone polyether 8 | Produit de laboratoire | 9 | 3,5 | 12,3 | 0,6 | H |
| Exemple de l'invention | Silicone polyether 9 | Produit de laboratoire | 48 | 6 | 15 | 5 | H |
| Exemple de l'invention | Silicone polyethers 10 | Rhodorsil SP3301 | 9,5 | 3,5 | 11,5 | 2,5 | H |

Le tableau II ci-dessous donne les résultats des mesures d'hydrophilie sur les films obtenus à partir des mélanges latex + silicones polyethers par la technique de mesure de l'angle de contact.

**Tableau II**

| | | | | | |
|---|---|---|---|---|---|
| | Référence des systèmes Latex + silicone polyether | | Angle de mouillage surface supérieure | Angle de mouillage surface inférieure | Résultat du Test |
| Témoin | Rhodopas D2040 seul | | 89° | 90° | |
| Exemple comparatif | Rhodopas +3%SP1 | D2040 | 55° | 85° | Négatif |
| Exemple comparatif | Rhodopas +3%SP2 | D2040 | 90° | 90° | Négatif |
| Exemple comparatif | Rhodopas +3%SP3 | D2040 | Pas de film obtenu | | Négatif |
| Exemple de l'invention | Rhodopas +3%SP4 | D2040 | 27° | 89° | Positif |
| Exemple de l'invention | Rhodopas +3%SP5 | D2040 | 27° | 90° | Positif |
| Exemple de l'invention | Rhodopas +3%SP6 | D2040 | 26° | 88° | Positif |
| Exemple de l'invention | Rhodopas +3%SP7 | D2040 | 25° | 87° | Positif |
| Exemple de l'invention | Rhodopas +3%SP8 | D2040 | 26° | 88° | Positif |
| Exemple de l'invention | Rhodopas +3%SP9 | D2040 | 26° | 90° | Positif |
| Exemple de l'invention | Rhodopas +3%SP10 | D2040 | 25° | 87° | Positif |

Ces résultats permettent de sélectionner nettement les silicones polyethers permettant d'obtenir l'hydrophilie de la face supérieure du film.

### Exemple 2 : Durabilité de l'effet obtenu

Comme décrit précédemment, les films ayant donné un résultat positif dans l'exemple précédent sont immergés et séchés 6 fois sur une durée d'une semaine, puis l'angle de contact est à nouveau mesuré sur les parties inférieures et supérieures du film. Les résultats figurent dans le tableau III ci-après :

**Tableau III**

| Référence des systèmes Latex + silicone polyther | | Angle de mouillage surface supérieure | Angle de mouillage surface inférieure | Angle de mouillage surface supérieure après test immersion | Angle de mouillage surface inférieure après test immersion |
|---|---|---|---|---|---|
| Rhodopas +3%SP4 | D2040 | 27° | 89° | 28° | 88° |
| Rhodopas +3%SP5 | D2040 | 27° | 90° | 27° | 90° |
| Rhodopas +3%SP6 | D2040 | 26° | 88° | 26° | 88° |
| Rhodopas +3%SP7 | D2040 | 25° | 87° | 27° | 87° |
| Rhodopas +3%SP8 | D2040 | 26° | 88° | 26° | 88° |
| Rhodopas +3%SP9 | D2040 | 26° | 90° | 27° | 87° |
| Rhodopas +3%SP10 | D2040 | 25° | 87° | 27° | 87° |

On constate que l'hydrophilie de la surface supérieure est parfaitement conservée après les cycles de trempage et de re-séchage. Elle est donc durable et résistante aux lessivages répétés.

### Exemple 3 - Etude de l'influence du dosage latex /silicone polyether selon l'invention

**L'exemple 1 a été réalisé avec des taux de silicone polyether de 3 %.** Les essais complémentaires ont porté sur des taux d'ajout de 1 %, 3 % et 5 % et 6 % du silicone polyether Rhodorsil SP3301 selon l'invention dans le latex Rhodopas D 2040, qui sont deux produits commercialisés par Rhodia.
La mesure des angles de contact avec l'eau distillée sur la partie supérieure du film montre qu'une certaine hydrophilie est atteinte dès un ajout de 1 %, et qu'elle augmente (diminution de l'angle de contact eau-surface) avec le taux de silicone polyether ajoutée :

| | |
|---|---|
| Pas d'ajout | 80° |
| 1 % | 50° |
| 3 % | 30° |
| 5 % | 30° |
| 6 % | 23° |
| 8 % | 20° |

Au-delà d'un ajout de 8 %, l'hydrophilie n'augmente plus.

### Exemple 4

### Hydrophilisation de certaines surfaces : cas d'une surface de polypropylène rigide

La plupart des matériaux peut être hydrophilisée en surface par ce système, à condition que le film adhère suffisamment sur la surface en question.
En particulier nous avons pu rendre hydrophile une surface de polypropylène rigide par application directe (par pinceau et par pulvérisation) d'un mélange de latex Rhodopas D 2040 et de 5 % de silicone polyether Rhodorsil SP3301.

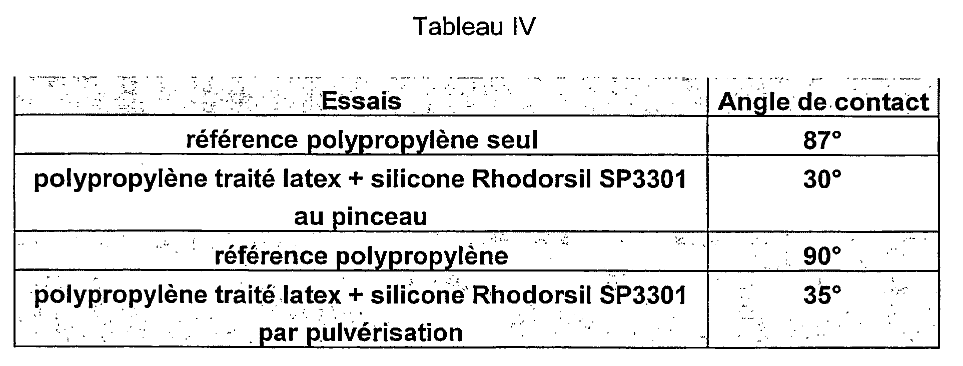

### Exemple 5

### Effet antisalissure. "Lavabilité" améliorée

Nous avons cherché à vérifier si le fait de rendre hydrophile une surface permet d'obtenir une meilleure lavabilité de cette surface. Ce fait est avéré dans le domaine des formulations lessivielles pour lavage du linge qui contiennent des polymères amphiphiles dits "anti-salissures" dont la fonction est d'hydrophiliser au lavage les textiles synthétiques notamment les polyesters.

Le matériau traité est du bois massif raboté, sur lequel on dépose une première couche de latex Rhodopas D2040, puis une couche de Rhodopas D2040 additivé de dose croissante de silicone polyether Rhodorsil SP 3301 selon le plan suivant présenté dans le tableau V :

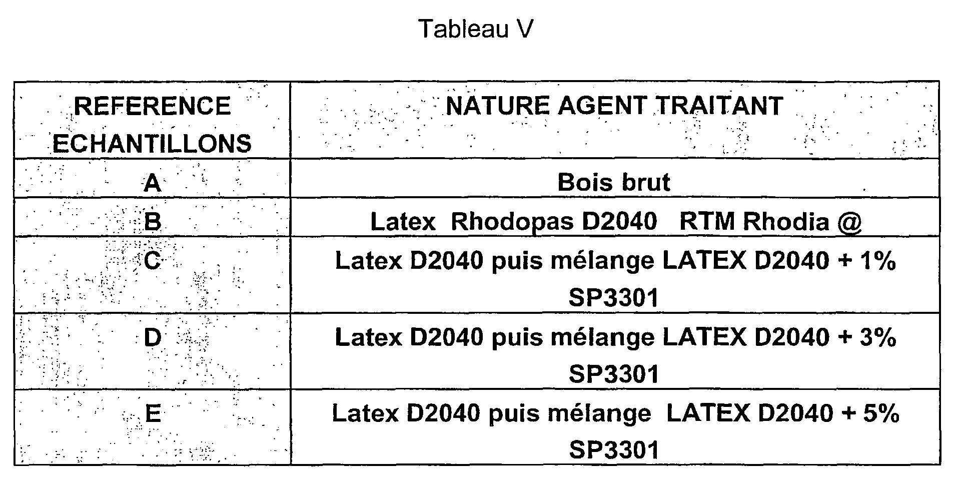

Ces éprouvettes de bois ainsi traitées ont ensuite été soumises à un ensalissement par divers types de salissures, par exemple de l'huile de vidange moteur (liquide).
Après ensalissement et séchage de 12 heures, les éprouvettes sont simplement passées sous un courant d'eau pour vérifier l'élimination de la salissure.

On note un effet positif et spectaculaire du traitement avec le latex additivé silicone polyether, comme le montrent les photographies de la **figure I**.

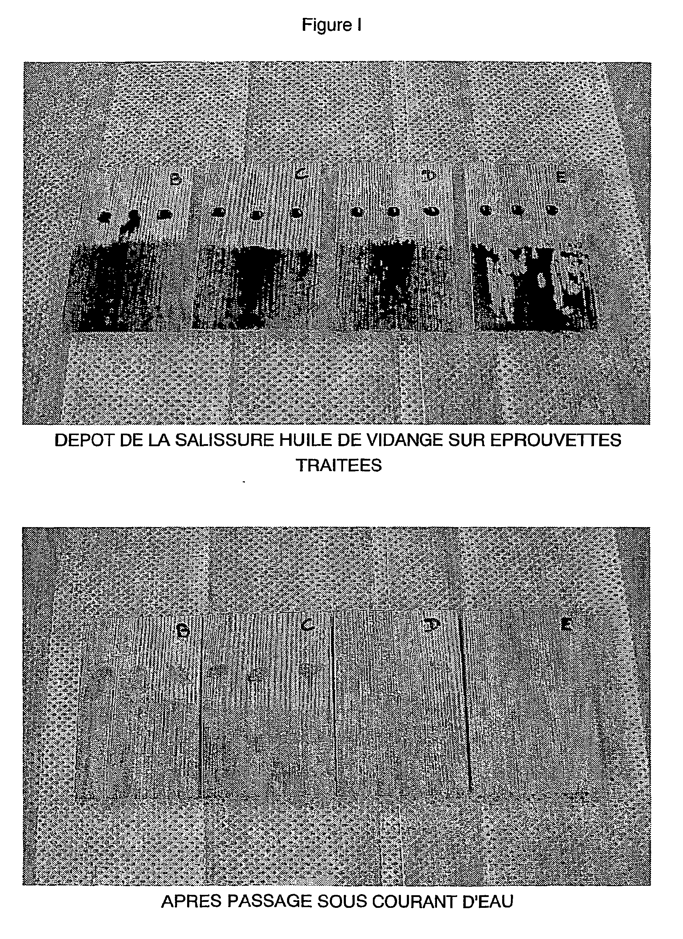

Avant même l'opération de lavage, on note que l'huile de vidange n'adhère pas sur la surface traitée par les mélanges contenant 3 % et 5 % de silicone polyether (démouillage visible sur la figure I).
Après passage sous l'eau, la salissure est beaucoup mieux éliminée sur les éprouvettes traitées, et totalement éliminée dans le cas de l'éprouvette traitée par un mélange contenant 5 % de silicone polyether.

## Revendications

1. Composition de revêtement comprenant :
- une dispersion aqueuse de polymère filmogène, et
- une quantité suffisante d'une silicone polyether vérifiant la formule (I) suivante : 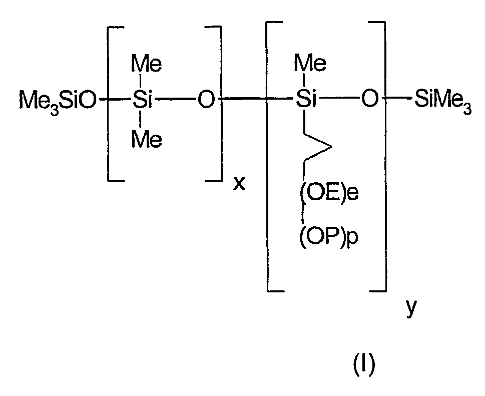 les groupes terminaux des oxydes d'éthylène ou de propylène étant des groupes OR
dans laquelle :
OE signifie -O-CH₂-CH₂-
OP signifie -O-CH₂-CH₂-CH₂-
R représente un atome d'hydrogène, ou un radical alkyle linéaire ou ramifié ayant de 1 à 22 atomes de carbone, et de préférence ayant de 1 à 4 atomes de carbone,
x est un nombre compris entre 5 et 50,
y est un nombre compris entre 3 et 10,
e est un nombre compris entre 10 et 30,
p est un nombre compris entre 0 et 10,
étant entendu que :
x/y est inférieur à 10 et de préférence inférieur ou égal à 8,
e+p est inférieur à 30 et de préférence inférieur ou égal à 20,
e/p est supérieur à 1 et de préférence supérieur ou égal à 4, et
x+y est inférieur à 60 et de préférence inférieur à 40.

2. Composition selon la revendication 1, **caractérisée en ce que** la silicone polyether est choisie parmi les silicones polyether de formule (I) vérifiant les conditions suivantes :
x= 9,5, y=3,5, e=11,5 et p=2,5, et R représente un atome d'hydrogène ;
x= 14, y= 4, e =17 et p= 1, et R représente un atome d'hydrogène ;
x= 48, y = 6 , e =15 et p = 5, et R représente un atome d'hydrogène.

3. Composition selon l'une des revendications 1 et 2, **caractérisée en ce que** la dispersion aqueuse de polymère filmogène (latex) comprend au moins un polymère insoluble dans l'eau obtenu par polymérisation de monomères choisis parmi :
- les esters vinyliques et plus particulièrement l'acétate de vinyle ;
- les acrylates et méthacrylates d'alkyle dont le groupe alkyle contient de 1 à 10 atomes de carbone par exemple les acrylates et méthacrylates de méthyle, éthyle, n-butyle, 2-éthylhexyle ;
- les monomères vinylaromatiques en particulier le styrène ;
ces monomères pouvant être copolymérisés entre eux ou avec d'autres monomères à insaturation éthylénique copolymérisables avec l'acétate de vinyle et/ou les esters acryliques et/ou le styrène, pour former des homopolymères, des copolymères ou des terpolymères.

4. Composition selon la revendication 3, **caractérisée en ce que** les monomères copolymérisables avec l'acétate de vinyle et/ou les esters acryliques et/ou le styrène sont choisis parmi l'éthylène et les oléfines comme l'isobutène; les esters vinyliques d'acides monocarboxyliques saturés, ramifiés ou non, ayant de 1 à 12 atomes de carbone, comme le propionate, le "Versatate" (marque déposée pour les esters d'acides ramifiés en C₉-C₁₁), le pivalate, le laurate de vinyle ; les esters d'acides insaturés mono- ou di-carboxyliques possédant 3 à 6 atomes de carbone avec les alcanols possédant 1 à 10 atomes de carbone, comme les maléates, fumarates de méthyle, d'éthyle, de butyle, d'éthylhexyle ; les monomères vinylaromatiques tels que les méthylstyrènes, les vinyltoluènes ; les halogénures de vinyle tels que le chlorure de vinyle, le chlorure de vinylidène, les diolefines particulièrement le butadiène ; les esters (méth)allyliques de l'acide (meth)acrylique, les esters (méth)allyliques des mono et diesters des acides maléique, fumarique et itaconique, ainsi que les dérivés alkèniques des amides des acides acryliques et méthacryliques, tels que le N-méthallylmaléimide.

5. Composition selon l'une des revendications 3 et 4, **caractérisée en ce que** la dispersion aqueuse de polymère filmogène (latex) comprend au moins un polymère insoluble dans l'eau obtenu par polymérisation de monomères choisis parmi les acrylates et méthacrylates d'alkyle dont le groupe alkyle contient de 1 à 10 atomes de carbone par exemple les acrylates et méthacrylates de méthyle, éthyle, n-butyle, 2-éthylhexyle.

6. Composition selon l'une des revendications 1 à 5, **caractérisée en ce que** la quantité suffisante de silicone polyether de formule (I) ajoutée dans la dispersion aqueuse de polymère filmogène (latex) est comprise entre 0,1 et 10 % en poids de silicone polyether de formule (I) sèche par rapport au poids de latex sec.

7. Composition selon la revendication 6, **caractérisée en ce que** la quantité suffisante de silicone polyether de formule (I) ajoutée dans la dispersion aqueuse de polymère filmogène (latex) est comprise entre 0,1 et 5 % en poids de silicone polyether de formule (I) sèche par rapport au poids de latex sec.

8. Procédé pour rendre hydrophile de façon durable un support hydrophobe **caractérisé en ce qu'**on applique sur la surface du support hydrophobe une quantité suffisante d'une composition de revêtement selon l'une des revendications 1 à 7.

9. Procédé selon la revendication 8, **caractérisé en ce que** le support hydrophobe a un angle de contact mesuré par la méthode de l'angle de mouillage supérieur à 70°.

10. Procédé selon l'une des revendications 8 et 9, **caractérisé en ce que** le support hydrophobe est choisi parmi le verre, les métaux, le polypropylène rigide, le bois traité par un vernis, ou un matériau à base de ciment préalablement traité par un primaire d'adhérence hydrophobe.

11. Procédé selon la revendication 10, **caractérisé en ce que** le primaire d'adhérence hydrophobe est une composition de polymère filmogène en dispersion aqueuse ou à base de solvant.

12. Procédé selon l'une des revendications 10 et 11, **caractérisé en ce que** le primaire d'adhérence est la dispersion aqueuse de polymère filmogène utilisée dans la composition de revêtement.

13. Support hydrophobe dont la surface est recouverte au moins en partie par un film provenant du séchage d'une composition comprenant une émulsion aqueuse de polymère filmogène et au moins une silicone polyether de formule (I) selon l'une des revendications 1 à 7.

14. Utilisation d'une composition de revêtement selon l'une des revendications 1 à 7 comme revêtement antisallisure.

15. Utilisation selon la revendication 14, **caractérisée en ce que** la salissure est choisie parmi les huiles, les suies, les mélanges d'huile et de particules de noir de carbone, les aérosols de fumées et de suies.

## Claims

1. Coating composition, comprising:
- an aqueous dispersion of film-forming polymer, and
- a sufficient amount of a silicone polyether satisfying formula (I) below: 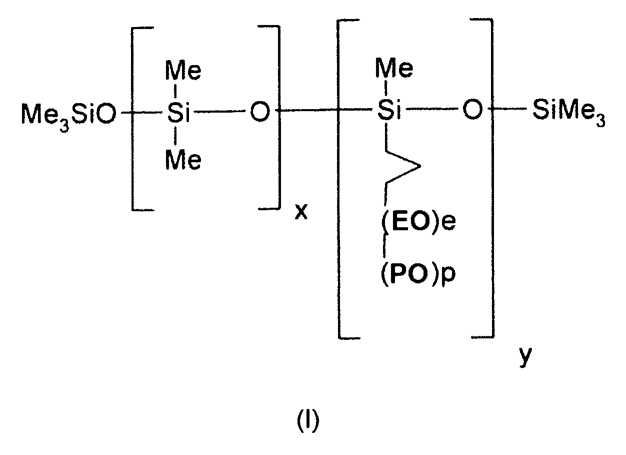 the terminal groups of the ethylene oxide or propylene oxide being OR groups
in which:
EO signifies -O-CH₂-CH₂-
PO signifies -O-CH₂-CH₂-CH₂-
R represents a hydrogen atom, or a linear or branched alkyl radical having from 1 to 22 carbon atoms, and preferably having from 1 to 4 carbon atoms,
x is a number between 5 and 50,
y is a number between 3 and 10,
e is a number between 10 and 30,
p is a number between 0 and 10,
it being understood that:
x/y is less than 10, and preferably less than or equal to 8,
e + p is less than 30, and preferably less than or equal to 20,
e/p is greater than 1, and preferably greater than or equal to 4, and
x + y is less than 60, and preferably less than 40.

2. Composition according to Claim 1, **characterized in that** the silicone polyether is chosen from the silicone polyethers of formula (I) satisfying the following conditions:
x = 9.5, y = 3.5, e = 11.5 and p = 2.5, and R is a hydrogen atom;
x = 14, y = 4, e = 17 and p = 1, and R is a hydrogen atom;
x = 48, y = 6, e = 15 and p = 5, and R is a hydrogen atom.

3. Composition according to either of Claims 1 and 2, **characterized in that** the aqueous dispersion of film-forming polymer (latex) comprises at least one water-insoluble polymer obtained by polymerization of monomers chosen from:
- vinyl esters, and more particularly vinyl acetate;
- alkyl acrylates and methacrylates in which the alkyl group contains from 1 to 10 carbon atoms, for example methyl acrylates and methacrylates, ethyl acrylates and methacrylates, n-butyl acrylates and methacrylates, and 2-ethylhexyl acrylates and methacrylates;
- vinylaromatic monomers, in particular styrene;
it being possible for these monomers to be copolymerized with one another or with other ethylenically unsaturated monomers copolymerizable with vinyl acetate and/or acrylic esters and/or styrene, so as to form homopolymers, copolymers or terpolymers.

4. Composition according to Claim 3, **characterized in that** the monomers copolymerizable with vinyl acetate and/or acrylic esters and/or styrene are chosen from ethylene and olefins such as isobutene; vinyl esters of branched or unbranched, saturated monocarboxylic acids having from 1 to 12 carbon atoms, such as vinyl propionate, vinyl "Versatate" (registered trade mark for esters of C₉-C₁₁ branched acids), vinyl pivalate, vinyl laurate; esters of unsaturated mono- or dicarboxylic acids having 3 to 6 carbon atoms with alkanols having 1 to 10 carbon atoms, such as methyl, ethyl, butyl or ethylhexyl maleates, or methyl, ethyl, butyl or ethylhexyl fumarates; vinylaromatic monomers such as methylstyrenes or vinyltoluenes; vinyl halides such as vinyl chloride, vinylidene chloride, diolefins, particularly butadiene; (meth)acrylic acid (meth)allyl esters, (meth)allyl esters of maleic acid mono- and diesters, fumaric acid mono- and diesters and itaconic acid mono- and diesters, and also alkene derivatives of acrylic and methacrylic acid amides, such as N-methallylmaleimide.

5. Composition according to either of Claims 3 and 4, **characterized in that** the aqueous dispersion of film-forming polymer (latex) comprises at least one water-insoluble polymer obtained by polymerization of monomers chosen from alkyl acrylates and methacrylates in which the alkyl group contains from 1 to 10 carbon atoms, for example methyl, ethyl, n-butyl or 2-ethylhexyl acrylates and methacrylates.

6. Composition according to one of Claims 1 to 5, **characterized in that** the sufficient amount of silicone polyether of formula (I) added to the aqueous dispersion of film-forming polymer (latex) is between 0.1 and 10% by weight of dry silicone polyether of formula (I) relative to the weight of dry latex.

7. Composition according to Claim 6, **characterized in that** the sufficient amount of silicone polyether of formula (I) added to the aqueous dispersion of film-forming polymer (latex) is between 0.1 and 5% by weight of dry silicone polyether of formula (I) relative to the weight of dry latex.

8. Process for rendering a hydrophobic support hydrophilic in a long-lasting manner, **characterized in that** a sufficient amount of a coating composition according to one of Claims 1 to 7 is applied to the surface of the hydrophobic support.

9. Process according to Claim 8, **characterized in that** the hydrophobic support has a contact angle measured by the wetting angle method of greater than 70°.

10. Process according to either of Claims 8 and 9, **characterized in that** the hydrophobic support is chosen from glass, metals, rigid polypropylene, wood treated with a varnish, or a cement-based material pretreated with a hydrophobic adhesion primer.

11. Process according to Claim 10, **characterized in that** the hydrophobic adhesion primer is a composition of film-forming polymer as an aqueous dispersion or that is solvent-based.

12. Process according to either of Claims 10 and 11, **characterized in that** the adhesion primer is the aqueous dispersion of film-forming polymer used in the coating composition.

13. Hydrophobic support whose surface is coated at least in part with a film resulting from the drying of a composition comprising an aqueous emulsion of film-forming polymer and at least one silicone polyether of formula (I) according to one of Claims 1 to 7.

14. Use of a coating composition according to one of Claims 1 to 7, as antisoiling coating.

15. Use according to Claim 14, **characterized in that** the soiling is chosen from oils, soot, mixtures of oil and carbon black particles, and smoke and soot aerosols.

## Patentansprüche

1. Überzugszusammensetzung, umfassend:
- eine wässrige Dispersion eines filmbildenden Polymer und
- eine hinreichende Menge eines Polyethersilicon der folgenden Formel (I): 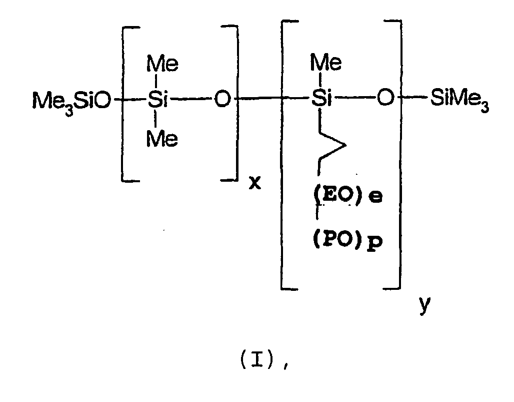
worin die Endgruppen der Ethylen- oder Propylenoxide OR-Gruppen sind und
worin ferner gilt:
EO bedeutet -O-CH₂-CH₂-;
PO bedeutet -O-CH₂-CH₂-CH₂-;
R stellt ein Wasserstoffatom oder einen linearen oder verzweigten Alkylrest mit 1 bis 22 und bevorzugt mit 1 bis 4 Kohlenstoffatomen dar;
x ist eine Zahl von 5 bis 50;
y ist eine Zahl von 3 bis 10;
e ist eine Zahl von 10 bis 30;
p ist eine Zahl von 0 bis 10;
x/y beträgt weniger als 10 und bevorzugt weniger oder gleich 8;
e+p beträgt weniger als 30 und bevorzugt weniger oder gleich 20;
e/p beträgt mehr als 1 und bevorzugt mehr oder gleich 4; und
x+y beträgt weniger als 60 und bevorzugt weniger als 40.

2. Zusammensetzung gemäß Anspruch 1, **dadurch gekennzeichnet, dass** das Polyethersilicon aus den Polyethersiliconen der Formel (I) ausgewählt ist, worin die folgenden Bedingungen gelten:
x = 9,5, y = 3,5, e = 11,5 und p = 2,5, und R stellt ein Wasserstoffatom dar;
x = 14, y = 4, e = 17 und p = 1, und R stellt ein Wasserstoffatom dar;
x = 48, y = 6, e = 15 und p = 5, und R stellt ein Wasserstoffatom dar.

3. Zusammensetzung gemäß einem der Ansprüche 1 und 2, **dadurch gekennzeichnet, dass** die wässrige Dispersion des filmbildenden Polymer (des Latex) mindestens 1 wasserunlösliches Polymer umfasst, das durch Polymerisation von Monomeren erhalten wird, die ausgewählt sind aus:
- Vinylestern und insbesondere aus Vinylacetat;
- Alkyl(meth)acrylaten, deren Alkylgruppe 1 bis 10 Kohlenstoffatome enthält, z.B. aus Methyl-, Ethyl-, n-Butyl- und 2-Ethylhexylacrylaten und -methacrylaten;
- vinylaromatischen Monomeren und insbesondere aus Styrol;
wobei diese Monomeren untereinander oder mit weiteren ethylenisch ungesättigten Monomeren copolymerisiert werden können, die mit Vinylacetat und/oder Acrylestern und/oder Styrol copolymerisierbar sind, um Homopolymere, Copolymere oder Terpolymere zu bilden.

4. Zusammensetzung gemäß Anspruch 3, **dadurch gekennzeichnet, dass** die mit Vinylacetat und/oder Acrylestern und/oder Styrol copolymerisierbaren Monomeren aus Ethylen und Olefinen wie Isobuten, Vinylestern gesättigter, verzweigter oder nichtverzweigter Monocarbonsäuren mit 1 bis 12 Kohlenstoffatomen, wie aus Vinylpropionat, Vinyl"versatat" (eingetragene Marke für die Ester verzweigter C₉₋₁₁-Säuren), Vinylpivalat und aus Vinyllaurat, Estern ungesättigter Mono- oder Dicarbonsäuren mit 3 bis 6 Kohlenstoffatomen mit Alkanolen mit 1 bis 10 Kohlenstoffatomen, wie aus Methyl-, Ethyl-, Butyl- und Ethylhexylmaleaten und -fumaraten, vinylaromatischen Monomeren, wie Methylstyrolen und Vinyltoluolen, Vinylhalogeniden, wie Vinylchlorid und Vinylidenchlorid, Olefinen, insbesondere aus Butadien, (Meth)allylestern von (Meth)acrylsäure, (Meth)allylestern aus Mono- und Diestern von Malein-, Fumar- und Itaconsäure sowie aus alkenischen Derivaten von Amiden von Acryl- und Methacrylsäure, wie aus N-Methallylmaleimid, ausgewählt sind.

5. Zusammensetzung gemäß einem der Ansprüche 3 und 4, **dadurch gekennzeichnet, dass** die wässrige Dispersion des filmbildenden Polymer (des Latex) mindestens ein wasserunlösliches Polymer umfasst, das durch Polymerisation von Monomeren erhalten wird, die aus Alkylacrylaten und -methacrylaten, deren Alkylgruppe 1 bis 10 Kohlenstoffatome enthält, z.B. aus Methyl-, Ethyl-, n-Butyl- und 2-Ethylhexylacrylaten und -methacrylaten, ausgewählt sind.

6. Zusammensetzung gemäß einem der Ansprüche 1 bis 5, **dadurch gekennzeichnet, dass** die hinreichende Menge des Polyethersilicon der Formel (I), die in die wässrige Dispersion des filmbildenden Polymer (des Latex) gegeben wird, 0,1 bis 10 Gew.-% trockenes Polyethersilicon der Formel (I) beträgt, bezogen auf das Gewicht des Trockenlatex.

7. Zusammensetzung gemäß Anspruch 6, **dadurch gekennzeichnet, dass** die hinreichende Menge des Polyethersilicon der Formel (I), die in die wässrige Dispersion des filmbildenden Polymer (des Latex) gegeben wird, 0,1 bis 5 Gew.-% trockenes Polyethersilicon der Formel (I) beträgt, bezogen auf das Gewicht des Trockenlatex.

8. Verfahren zur dauerhaften Hydrophilierung einer hydrophoben Unterlage, **dadurch gekennzeichnet, dass** man auf die Oberfläche der hydrophoben Unterlage eine hinreichende Menge einer Überzugszusammensetzung gemäß einem der Ansprüche 1 bis 7 aufbringt.

9. Verfahren gemäß Anspruch 8, **dadurch gekennzeichnet, dass** die hydrophobe Unterlage einen mit der Befeuchtungswinkelmethode gemessenen Kontaktwinkel von mehr als 70° aufweist.

10. Verfahren gemäß einem der Ansprüche 8 und 9, **dadurch gekennzeichnet, dass** die hydrophobe Unterlage aus Glas, Metallen, Hartpolypropylen, mit Lack behandeltem Holz oder aus einem Material auf Basis von Zement, welche vorab mit einer hydrophoben Haftgrundierung behandelt worden sind, ausgewählt ist.

11. Verfahren gemäß Anspruch 10, **dadurch gekennzeichnet, dass** die hydrophobe Haftgrundierung eine Zusammensetzung des filmbildenden Polymer in wässriger Dispersion oder auf Basis eines Lösungsmittels ist.

12. Verfahren gemäß einem der Ansprüche 10 und 11, **dadurch gekennzeichnet, dass** die Haftgrundierung die wässrige Dispersion des in der Überzugszusammensetzung verwendeten filmbildenden Polymer ist.

13. Hydrophobe Unterlage, deren Oberfläche zumindest teilweise mit einem Film bedeckt ist, der aus der Trocknung einer Zusammensetzung stammt, die die wässrige Emulsion gemäß einem der Ansprüche 1 bis 7 des filmbildenden Polymer und mindestens des Polyethersilicon der Formel (I) umfasst.

14. Verwendung einer Überzugszusammensetzung gemäß einem der Ansprüche 1 bis 7 als Antischmutzüberzug.

15. Verwendung gemäß Anspruch 14, **dadurch gekennzeichnet, dass** die Verschmutzung aus Ölen, Rußen, Mischungen aus Öl und Rußpartikeln sowie aus Rauch- und Rußaerosolen ausgewählt ist.
